# EUROPEAN PATENT APPLICATION

(11) **EP 0 590 698 A2**
(43) Date of publication of application: **06.04.1994**
(21) Application number: 93201557.1
(22) Date of filing: 01.06.1993
(51) Int. Cl.: A61F 11/08, H04R 25/00, G10K 11/16

(54) **Hearing protector**

(30) Priority: 31.05.1992 NL 9200957
(71) Applicant: Verenigde Bedrijven Groeneveld B.V., NL-3316 GB Dordrecht (NL)
(72) Inventor: Labrijn, Hans, 3334 AG Zwijndrecht (NL)
(74) Representative: Boelsma, Gerben Harm, Ir.

(57) **Abstract**

The invention relates to a hearing protector of the type, comprising a housing (1) having a receptacle portion (2) to be applied against the auricle and a portion (3) projecting therefrom for fitting into the auditory canal, an outwardly opening chamber (5) being provided in said receptacle portion (2) to receive an acoustic filter (7), said chamber (5) being connected to a passage (6) that extends through said portion (3) fitting in the auditory canal and emanates at the free end of the latter portion (3), means (9) being provided for the connection of a miniature loud-speaker (10). The means (9) for connecting a miniature loud-speaker (10) are provided in the respective chamber (5) at a location upstream of said acoustic filter (7).

## Description

The invention relates to a hearing protector of the type, comprising a housing having a receptacle portion to be applied against the auricle and a portion projecting therefrom for fitting into the auditory canal, an outwardly opening chamber being provided in said receptacle portion to receive an acoustic filter, said chamber being connected to a passage that extends through said portion fitting in the auditory canal and emanates at the free end of the latter portion, means being provided for the connection of a miniature loud-speaker.

Such hearing protectors, in professional circles called "oto-plastique", are well-known. They are primarily intended to receive an acoustic filter for damping the harmful frequencies of the entering sound. Furthermore a connection to a communication system, such as a calling system or similar system, is often desired. For this purpose the hearing protector is provided with means for the connection of a miniature emitter or loud-speaker making part of such communication system.

In the well-known embodiments of such a hearing protector the means for connecting a miniature loud-speaker is formed by a separate passage extending through the receptable portion and the auditory channel portion, a snap ring being often provided in a widened mouth of said passage at the outer side of the receptacle portion for locking the minature loud-speaker according to the push button principle. The loud-speaker passage may be closed by a plug in case no miniature loud-speaker is to be connected. Reference is made to EP-A-0333298, which discloses such a hearing protector having a second passage, in which, however, the second passage is intended for testing purposes rather than for the connection of a miniature loud-speaker.

A drawback of such hearing protectors is, that in general the connection of the miniature loud-speakers is not sound-proof. This means that the sound leakage through the loud-speaker passage will substantially affect the sound damping effect of the acoustic filter.

The present invention provides a simple and effective solution for this problem, said solution being characterized in that the means for connecting a miniature loud-speaker are provided in the respective chamber at a location upstream of said acoustic filter. With a hearing protector constructed in accordance with the proposal of the invention a sound leakage in the connection of and via a miniature loud-speaker has no harmful effect anymore, because the leaking sound is going through the acoustic filter and consequently will be freed from its harmful frequencies by this filter. Consequently, with the hearing protector according to the invention an imperfectly connecting miniature loud-speaker or a miniature loud-speaker of minor quality will not damage the hearing protection.

The proposal according to the invention also means an essential simplification of the hearing protector due to the omission of the second passage, whereas it is also advantageous, that no plug needs to be applied in case no miniature loud-speaker is to be connected.

In a practical embodiment, in which - like the well-known embodiment - a snap ring is used for connecting a miniature loud-speaker, such snap ring is provided in the mouth of the acoustic filter.

A particular embodiment of a hearing protector with a snap ring for connecting a miniature loud-speaker, is characterized in that the snap ring is integrally formed with a component of the acoustic filter.

Two embodiments of the invention will be hereinafter further explained by reference to the drawing.
Fig. 1 shows a cross-section through a hearing protector according to the invention in a first embodiment and
Fig. 2 shows a cross-section through a hearing protector according to the invention in a second embodiment.

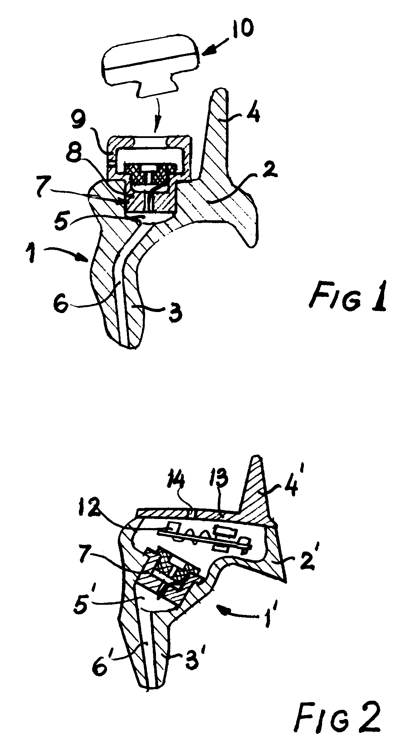

With reference to fig. 1, the hearing protector according to the invention comprises a housing 1 of a suitable plastics material, such as pmma, consisting of a receptacle portion 2 and a portion 3 projecting therefrom. The portion 3 fits in the auditory canal of the user, whereas in use the receptacle portion will be applied against the auricle of the user. A finger grip 4 is provided on the outer side of the receptacle portion. In the receptacle portion 2 there is an outwardly opening chamber 5, which is connected to the free end of the portion 3 by means of a passage 6 extending through said portion 3. The chamber 5 is adapted to accommodate an acoustic filter, such as the filter designated at 7 in fig. 1. This filter is of a well-known type and will therefore not be described in detail. In accordance with the invention the outer part 8 of the filter is provided with an integrally moulded snap ring 9, which is adapted to constitute a push button connection with a well-known miniature loud-speaker, indicated at 10. The connection of this miniature loud-speaker may be simply effected by pressing its push button portion in the arrow direction into the opening of the snap ring 9. In fact the embodiment of fig. 1 corresponds with a hearing protector of a well-known type, which has - by a simple modification of the acoustic filter, i.e. by providing the outer part of it with a snap ring - become suitable for use in combination with communication facilities.

In the embodiment of fig. 2 a modified housing 1' is used, in which the chamber 5' for accommodating the acoustic filter 7' is located further inwardly, whereas adjacent the outer side of the receptacle portion 2' a space 11 is formed, in which a well-known miniature emitter-receiver 12 is provided. The space 11 is closed by a cover 13 which carries the finger grip 4' and is provided with an opening 14.

Unlike the embodiment of fig. 1, the acoustic filter in the embodiment of fig. 2 has not been changed, while the housing 1' is modified for accommodating a miniature emitter-receiver.

## Claims

1. A hearing protector of the type, comprising a housing having a receptacle portion to be applied against the auricle and a portion projecting therefrom for fitting into the auditory canal, an outwardly opening chamber being provided in said receptacle portion to receive an acoustic filter, said chamber being connected to a passage that extends through said portion fitting in the auditory canal and emanates at the free end of the latter portion, means being provided for the connection of a miniature loud-speaker, characterized in that the means for connecting a miniature loud-speaker are provided in the respective chamber at a location upstream of said acoustic filter.

2. A hearing protector according to claim 1, wherein a snap ring is used for connecting a miniature loud-speaker, characterized in that said snap ring is provided in the mouth of the acoustic filter at a location upstream of said acoustic filter.

3. A hearing protector according to claim 2, characterized in that the snap ring is integrally formed with a component of the acoustic filter.

4. A hearing protector according to claim 1, characterized in that the chamber with the acoustic filter is located further inwardly in said receptacle portion and that in the space created thereby the upstream side of the acoustic filter a miniature emitter-received is provided, of which the loud-speaker is making a part, said space being outwardly closed by a cover in which a central vent is provided.
